# EUROPEAN PATENT SPECIFICATION

(11) **EP 1 983 899 B1**
(45) Date of publication and mention of the grant of the patent: **27.10.2010**
(21) Application number: 07705480.7
(22) Date of filing: 26.01.2007
(51) Int. Cl.: A61B 8/12, A61B 10/02, F16M 11/14, A61B 8/08, A61B 19/00

(54) **APPARATUS FOR MOTORISED PLACEMENT OF NEEDLE**
VORRICHTUNG ZUR MOTORISIERTEN NADELPLATZIERUNG
APPAREIL DE MISE EN PLACE MOTORISÉE D'UNE AIGUILLE

(30) Priority: 26.01.2006 US 762126 P
(43) Date of publication of application: 29.10.2008
(73) Proprietor: Nanyang Technological University, Singapore 639798 (SG); Singapore Health Services Pte Ltd, Singapore 168751 (SG)
(72) Inventor: CHENG, Wai Sam Christopher, Singapore 807354 (SG); NG, Wan Sing, Singapore 678948 (SG)
(74) Representative: Walton, Seán Malcolm
(86) International application number: PCT/IB2007/000194
(87) International publication number: WO 2007/085953

(56) References cited:
- EP-A1- 0 654 244
- WO-A1-00/30557
- US-A1- 2002 156 376
- DI XIAO ET AL: "Software design of transperineal prostate needle biopsy robot" CONTROL APPLICATIONS, 2005. CCA 2005. PROCEEDINGS OF 2005 IEEE CONFERENCE ON TORONTO, CANADA AUG. 29-31, 2005, PISCATAWAY, NJ, USA,IEEE, 29 August 2005 (2005-08-29), pages 13-18, XP010835053 ISBN: 0-7803-9354-6
- NG W S ET AL: "ROBOTIC SURGERY. ÖA FIRST-HAND EXPERIENCE IN TRANSURETHRAL RESECTION OF THE PROSTATE" IEEE ENGINEERING IN MEDICINE AND BIOLOGY MAGAZINE, IEEE SERVICE CENTER, PISACATAWAY, NJ, US, vol. 12, no. 1, 1 March 1993 (1993-03-01), pages 120-125, XP000345180 ISSN: 0739-5175
- PHEE L ET AL: "Ultrasound Guided Robotic System for Transperineal Biopsy of the Prostate" ROBOTICS AND AUTOMATION, 2005. PROCEEDINGS OF THE 2005 IEEE INTERNATIONAL CONFERENCE ON BARCELONA, SPAIN 18-22 APRIL 2005, PISCATAWAY, NJ, USA,IEEE, 18 April 2005 (2005-04-18), pages 1315-1320, XP010871859 ISBN: 0-7803-8914-X cited in the application

## Description

### FIELD OF THE INVENTION

The present invention provides an apparatus to insert a needle through a predetermined trajectory and with a predetermined depth, in order to reach a target accurately within soft tissue, under ultrasound imaging guidance. The invention can be applied to carry out, for example, prostate biopsy, or prostate brachytherapy.

### BACKGROUND OF THE INVENTION

A prostate needle biopsy is recommended when prostate cancer is suspected. It is a surgical procedure in which a small sample of tissue is removed from the prostate gland and examined under the microscope by a pathologist, a doctor specializing in identifying disease through the study of cells, tissues and organs. The procedure takes about 15 minutes and is usually performed by an urologist with the use of a transrectal ultrasound (TRUS) probe. No anaesthetic is required. With the help of TRUS, a doctor guides a biopsy gun - a hand-held device with a spring-loaded, slender needle - through the wall of the rectum into the area of the prostate gland that appears abnormal.

The rectal wall is thin, so it is possible to place the needle accurately into the abnormal site of the prostate gland with the use of a biopsy gun with less injury to other tissues. When the biopsy gun is activated, the needle can remove a slender cylinder of tissue (about 1/2" (1.27cm) by 1/16" (0.159cm)), called a core, in a fraction of a second. Biopsy needles are tiny-only 1.2 millimeters in diameter and less than 1/2" (1.27cm) long. A sliding sheath on the gun opens once the needle enters the prostate, closes onto a sample of tissue and the needle is withdrawn. A sextant (six-part) biopsy is the most common prostate biopsy procedure. An average of six cores are taken from the prostate (top, middle and bottom; right and left sides) to get a representative sample of the prostate gland and to determine the extent of any cancer.

However, the current practice for prostate biopsies has the following shortfalls:
1. Random biopsy sites: The maximum total volume of tissue samples retrieved can be calculated to be, for example, about 220 mm³ in a 14-core method and the volume size of a typical prostate with a diameter of 40mm is about 33,500 mm³. Therefore, the biopsy cores represent only about 0.6% of the prostate in terms of volume. Without having accurate knowledge of the cancer site(s), it is unlikely that a random biopsy protocol will yield consistently high cancer detection rates.
2. Inaccurate needling: Although the biopsy is guided by TRUS, it is known that the biopsy needle may not reach the desired position accurately and quickly under manual control. Depending on the skills and experience of the urologist, inaccuracies in terms of centimeters are common occurrences.
3. Limited 2D guidance: The TRUS used for biopsy guidance is 2D ultrasound images. As the biopsy sites are distributed in 3D, it is difficult for the surgeon to imagine the overall picture of the biopsy sites and to identify the biopsy site accurately and intuitively.
4. Transrectal: As most of the cancer occurs at the apex area of the prostate, a transperineal biopsy is considered to have a higher chance of obtaining cancer tissue, compared with the conventional transrectal biopsy. Furthermore, a transperineal biopsy is considered "cleaner", as its puncture point is on the skin, rather than on the rectum.

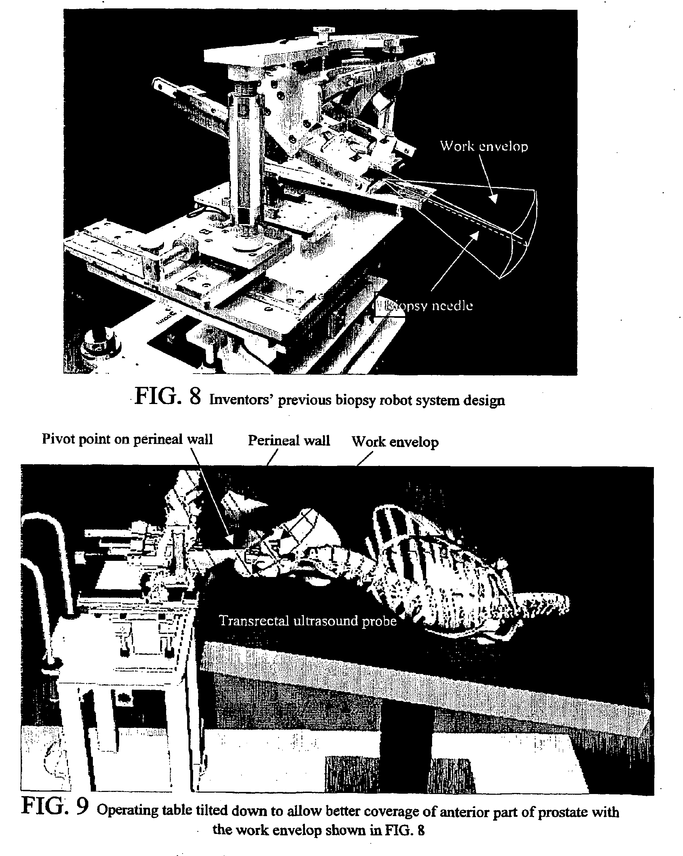

The inventors have previously developed a biopsy robot system to try to address the problems above (see FIG 8 and FIG 9, L Phee, D Xiao, WS Ng et al, "Ultrasound guided robotic system for transperineal biopsy of the prostate", IEEE International Conference on Robotics and Automation, Barcelona, Spain, May 2005.). This biopsy robot system has a gantry mechanism to assist the needling. The gantry holds the biopsy gun, and is manually driven but has markers on it, which the user can follow to help position the needle. Several clinical trials have proven that the needle can consistently reach the specified target with ±2mm error. In order to have a full picture of the prostate and plan in 3D, the previous biopsy robot system drives the 2D ultrasound probe to obtain a series of 2D images, and allows the planning to be done in 3D space. The 3D visualization helps the surgeon plan the biopsy site more intuitively. 3D ultrasound scanners are available but are more expensive and less common, therefore 2D image stacks are used rather than 3D images. This biopsy robot system utilizes the transperineal approach to achieve a cleaner biopsy and covers the apex area of the prostate better.

However, the biopsy robot system described above is mainly manually operated, except for the positioning of the ultrasound probe, which is motor driven. It uses the lower half of a single cone to reach the prostate in order to avoid the confliction between the biopsy gun and the ultrasound probe. With this approach, the needle may not reach the upper region of the prostate due to pubic bone interference and inherent truncated conical work envelop as shown in FIG 8. Furthermore the system shown in FIG 8 is unable to offer multiple pivot points for needle entry.

The inventors have now developed the present invention which is fully motorised with improvements to address the problems of current prostate biopsy systems.

### DESCRIPTION OF FIGURES

FIG 1 is a sectional view showing a conical approach of reaching the prostate through the perineal wall.

FIG 2 is an overview of a preferred embodiment.

FIG 3 is a closeup view of a needling module and imaging module mounted on the 5 DOF platform.

FIG 4 is a side view of a needling module and imaging module with patient showing needle entry and its relationship with the ultrasound probe.

FIG 5 is an isometric view of a needling module and an imaging module of a preferred embodiment and a pseudo prostate in approximate location with respect to the probe and needle

FIG 6 is a front view (6A) and a back view (6B) of a needle sheath orientation assembly in the needle module of a preferred embodiment.

FIG 7 is a side view (7A) and bottom view (7B) of a biopsy gun holder with motorized stopper in a preferred embodiment.

### DESCRIPTION OF THE INVENTION

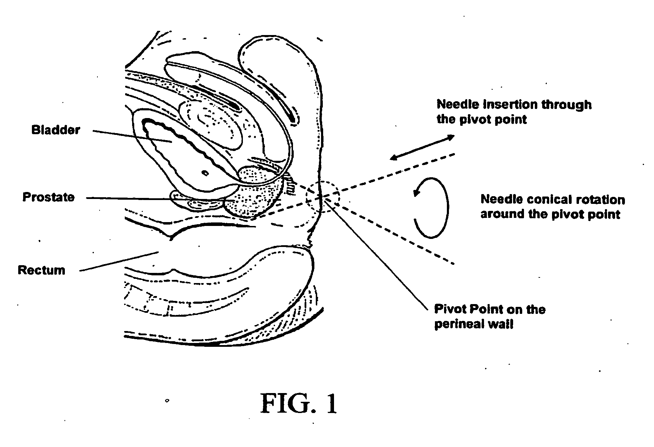

The present invention provides an apparatus for enabling a percutaneous needle to be inserted accurately to predefined points within soft tissue. As shown in FIG 1, a conical approach (that is, a path within one or more conical envelopes) is used to reach the prostate through one or more pivot points (thus providing more than one cone) on the perineal wall. The biopsy needle penetrates the patient's skin through a pivot point for multiple cores. The needle may conically rotate around the pivot point. Multiple pivot points may be necessary in the case that the pubic bone blocks the needle's way to some part of the prostate.

The apparatus comprises of two important mechanical modules: a needling module and an imaging module. They are mounted on a 5 degrees of freedom (DOF) platform, which is in turn may be mounted on a mobile cart. The system also includes an industrial PC, a touch screen, related accessories, and electrical equipment.

The needling module orients a needle sheath within a conical shape, with the tip of the cone as the pivot point, which is defined by the surgeon on the perineal wall. The biopsy needle is inserted manually through the needle sheath. The depth of the insertion is controlled by the position of the stopper on the biopsy gun holder. Both the orientation and stopper depth are driven by motors.

The imaging module consists of an ultrasound probe sheath, a trans-rectal ultrasound probe, and an ultrasound probe holder which can translate the probe using a motor. Volumetric image data is constructed by obtaining a stack of 2D ultrasound images. Target locations are determined in the volumetric image data by the surgeon based on experience and/or meaningful patient medical information. Positions in the image data are mapped to physical coordinates via a calibration method, which will later be used to compute the orientation and depth for the needling module. The ultrasound probe sheath prevents the moving ultrasound probe from disturbing the prostate, thus reducing target movement.

The 5 DOF platform gives the surgeon improved dexterity sufficient to position the imaging module and needling module with respect to the patient. During the imaging and needling procedure, the ultrasound probe sheath of the imaging module and the pivot point of the needling module are maintained fixed with respect to this supporting platform.

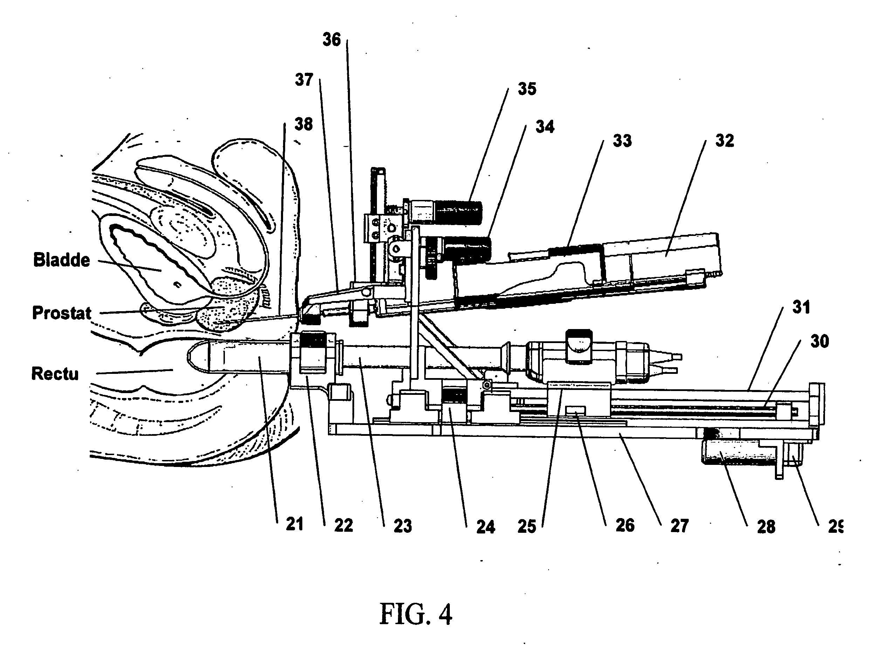

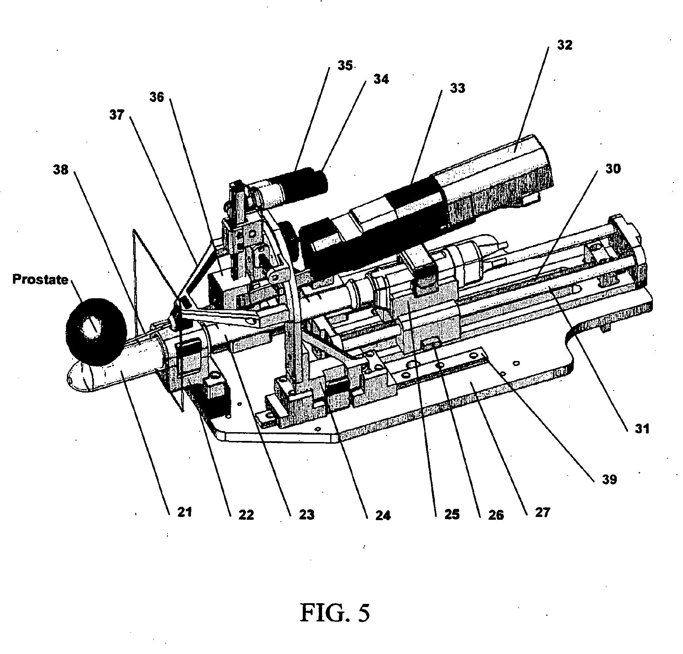

The ultrasound probe holder 25 (FIG 4 and FIG 5) has a known spatial relationship to the needling gantry 24 (FIG 4 and FIG 5) because they share a common reference on platform 27. The movement of the gantry is measured by a position sensor (not shown in either FIG 4 or FIG 5), which may be mounted below platform 27, that of the ultrasound probe is measured by a position sensor, which may mounted at the rear of the motor 28.

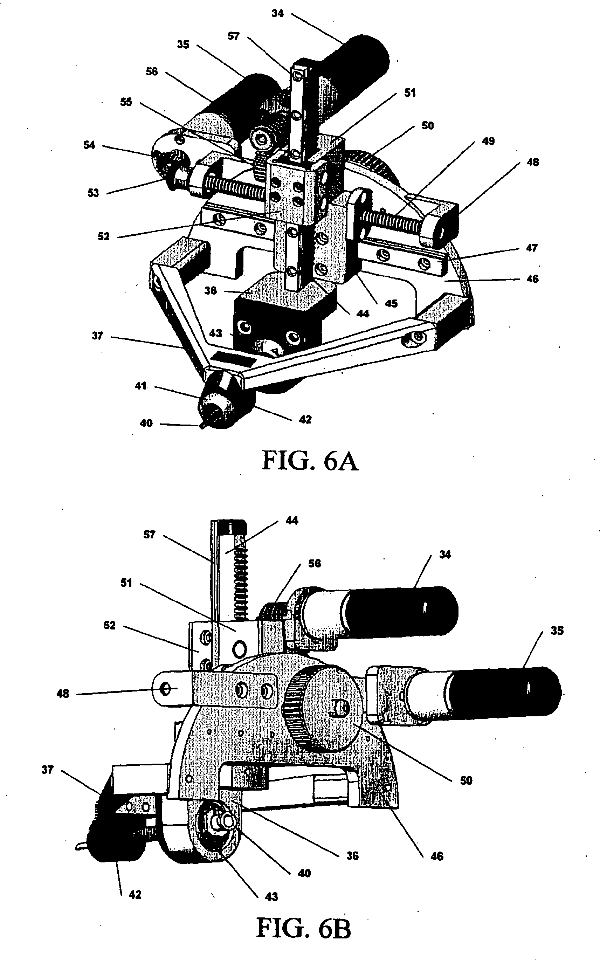

The gun stopper 58 is moved to a pre-calculated position and its tip is pushed up against the back of the needle sheath 40 (FIG 6) during use. This will give the desired needle depth as the user drives the needle together with the gun holder (64)/stopper (58) until it cannot move further. The gun is now fired to obtain a piece of tissue sample or to place a brachytherapy seed, etc.

the invention provides a needle guide apparatus for guiding a needle into a selected location of a patient relative to an imaging instrument, for percutaneous interventional procedures like prostate biopsy and brachytherapy. The apparatus comprises 1) a needling module to hold and orientate the needle sheath and control the insertion depth by motors, wherein the means for holding the needle sheath orientation assembly includes two ball joints that allow different pivot points to be defined and resulting conical envelops can avoid pubic arch interference and the urethra, 2) an imaging module to hold and position the ultrasound probe without disturbing the prostate; and 3) a 5 DOF supporting platform to support the needling module and imaging module. Using such an apparatus, a needle is inserted to reach a target accurately within soft tissue through a predetermined trajectory and with a predetermined depth under ultrasound imaging guidance.

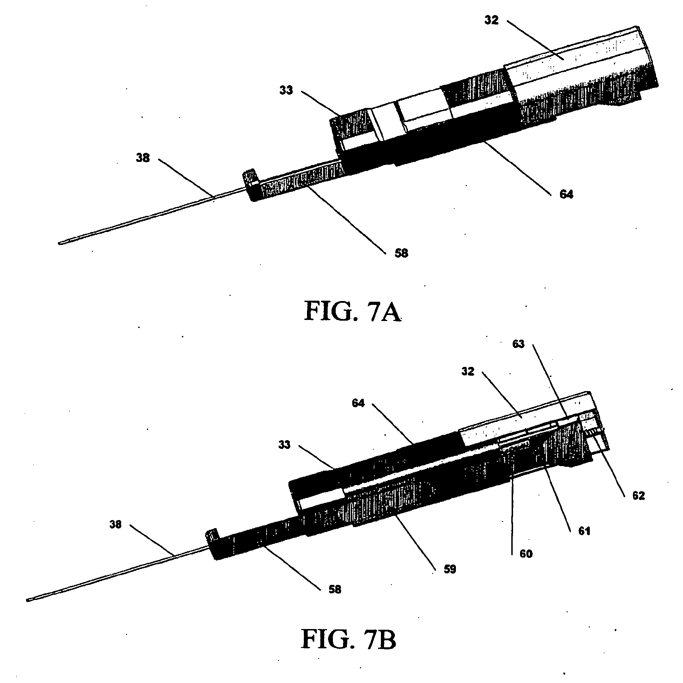

Comparing with the inventors' previous biopsy robot system design with the present invention, there are four major differences:
1. Double ball joints are used in the present invention to achieve the conical orientation of the needle sheath. The front ball joint which represents the pivot point is fixed, while the back ball joint may move in a virtual plane to orient the needle sheath. The previous design used a gantry with large curve sliding guides, which takes up much more space. Furthermore, the present invention is mostly motor driven while the previous design is mainly manually operated
2. In the present invention, the user may change the mounting point of the whole needle sheath orientation assembly to define different pivot points. As mentioned before, multiple pivot point is necessary in case the pubic bone blocks the needle's way to the prostate. Rather than having one pivot point in the midline of the patient, the present invention offers two or more pivot points on each side of the midline/midplane. When implementing conical needling on these pivot points, the resulting geometry not only can avoid the urethra but also the pubic arch. It also provides good coverage of the prostate as a whole as well as being capable of optimising coverage of the peripheral zone of the prostate where cancer is statistically known to reside.
3. When inserting the needle, the biopsy gun of the present invention is held by the surgeon manually rather than being mounted on a sliding platform. The previous design required the biopsy gun to be mounted on a sliding platform. It is considered that the needle sheath is sufficient to guarantee the trajectory of the needle. The present invention eliminates the procedure of mounting and dismounting the biopsy gun, thus speeding up the procedure. The biopsy gun in the present invention is attached to a motor and a set of transmissions (FIG 7) providing a stopper to control the depth of insertion of a needle Similar design could be done for different biopsy guns. Alternatively, a customized biopsy gun could be made to incorporate the stopper and other additional needle motions such as rotation and forward-backward oscillation.
4. The present invention has means to prevent prostate movement. It has been observed that a moving ultrasound probe inside the rectum may disturb the prostate and move or deform it. The new design incorporates an ultrasound probe sheath preferably made of TPX (4_methyl-1 pentene) materials. It has been proved that the probe sheath does not have any significant side effect on the ultrasound image quality.

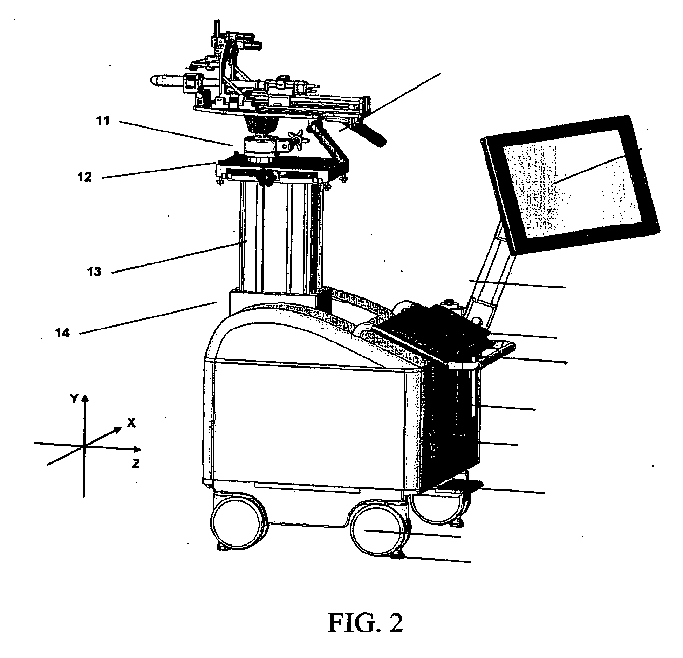

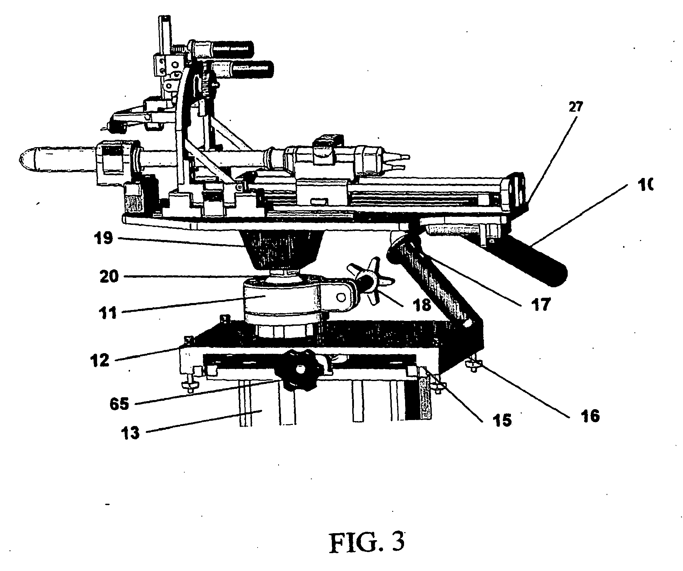

FIG 2 shows the overview of a preferred embodiment of the present invention. The mobile cart 4 has four wheels 2 under it and each wheel has a standing pole 1, which can be lowered to lock the cart 4 when the paddle 3 is pressed down. Guaranteeing a firm lock for the cart is important as the procedure assumes the cart is fixed with respect to the patient. The cart 4 hosts the controller PC 5 and other electrical equipments. The cart 4 also hosts a linear guide (not shown in the figure) on which the up-and-down lift (13 & 14) may slide along the Z axis. The lift is driven by motors so that the inner part 13 can go up and down inside the outer part 14. The up-and-down motor is controlled by the switch 17 (FIG 3) on the handle 10.

FIG 3 gives a closer look on the top of the 5 DOF platform. A table 12 is mounted to the top of lift part 13 via a pair of linear guides 15 and may slide along the X axis. The table 12 is lockable by manually tightening the knob 65. Ball joint (20 & 11) gives the upper part three more DOF, and is lockable by manually tightening the knob 18. Adaptor 19 connects the upper parts of the needling module to the ball joint 20. The handle 10 is for the user to adjust the posture of the ball joint 20. There are also four springs. (not shown in the figure) that link the table 12 to the upper platform 27 via four pairs of fitness 16.

FIG 4 illustrates the needling module and imaging module of a preferred embodiment being applied to a patient. The surgeon positions the ultrasound probe sheath 21 within the patient's rectum, so that the ultrasound probe 23 can cover the whole prostate when translating within the sheath. Then the supporting platform 27 is adjusted by controlling the up-and-down lift 13, the table 12, and ball joint 20, until the probe sheath holder 22 matches the probe sheath 21, and the surgeon fixes the sheath 21 to sheath holder 22. After that, the ultrasound probe 23 is mounted to the probe holder 25, which can be moved manually or driven by motor 28, via gear box 29, ball screw 30 and linear guide 31. Button 26 is to release the clutch within the probe holder 25, which in turn disengages the motor transmission.

As shown in FIG 4 and FIG 5, needling gantry 24 may translate along the sliding guide 39. The whole needle sheath orientation assembly (FIG 6) is mounted to this needling gantry 24 at a pre-defined mounting point by tightening the big screw 50. The surgeon pushes the needling gantry 24 until the front ball joint 41 (FIG 6) touches the patient's perineal wall. The touching point is defined as the pivot point. Changing the mounting point of the needle sheath orientation assembly on the needling gantry 24 allows different pivot points to be defined. Once the pivot point is defined, the needling gantry 24 is locked with respect to the supporting platform 27. The front ball joint 41 is in turn locked, due to the rigid connection with the needling gantry 24 via screw 50, horse shoe shape 46, bridge 37, and holder 42, as shown in FIG 5 and FIG 6. Assuming that the patient does not move with respect to the supporting platform 27, the front ball joint 41 is considered fixed with respect to the patient, representing the pivot point on the perineal wall.

FIG 6 shows the details of the needle sheath orientation assembly. The needle sheath 40 goes through two ball joints 41 and 43. As mentioned before, the front ball joint 41 is considered the pivot point. The orientation of the needle sheath 40 is achieved by moving the back ball joint 43 on a virtual plane. (Note the X-Y plane defined by ball screw 49 and linear guide 57.) The ball joint 43 is hosted by holder 36. The ball joint 41, its holder 42, and ball joint 43 are components that are detachable for sterilization purpose. By changing the inner dimension of the two ball joints 41 and 43, different sizes of needle sheath 40 can be used. The holder 36 may move up and down by motor 34, via worm gear 56, worm wheel 55, gear box 51, rack 44, linear guide 57, and slide 52. The gear box 51 is mounted on the slide 45. Left and right movement of the holder 36 is achieved by moving the gear box 51 horizontally by motor 35, via bevel gears 53 and 54, ball screw 49, slide 45, and linear guide 47.

FIG 7 illustrates the biopsy gun holder with a motorized stopper. The biopsy gun 33 is hosted by holder 64, with its needle 38 going through the stopper 58. The stopper 58 may be positioned at various positions along the needle, driven by motor 60, via ball screw 62, slider 63 (with screw nut), and linear guide 61. Cover 59 is the surgeon's holding position. This portion may be replaced by a customized biopsy gun with needle rotation motion or forward-backward oscillation, or in the same manner, a compact brachytherapy seed applicator, with or without needle rotation/oscillation.

## Claims

1. A needle guide apparatus for guiding a needle (38) into a selected location of a patient relative to an imaging instrument (23), for percutaneous interventional procedures, comprising:
a needling module comprising a needle sheath holder (36, 42) and orientation assembly that comprises two ball joints (41, 43) providing different pivot points for a needle held in the needle sheath holder and resulting in conical motion envelopes for said needle; and
an imaging module that holds and positions an ultrasound probe (23),
said needling module and said imaging module being mounted fixedly upon a 5 DOF supporting platform (27).

2. The needle guide apparatus of claim 1, in which the needling module further comprises a gun stopper (68) that sets a desired needle depth and is set to a pre-calculated position based upon imaging data from the ultrasound probe.

3. The needle guide apparatus of claim 1, wherein the imaging module includes an ultrasound probe sheath (21) an ultrasound probe, and an ultrasound probe holder adapted to translate the probe using a motor.

## Patentansprüche

1. Nadelführungsvorrichtung zur Führung einer Nadel (38) in eine ausgewählte Stelle eines Patienten relativ zu einem Bildgebungsinstrument (23) für perkutane Eingriffe, wobei die Vorrichtung Folgendes umfasst:
ein Nadelmodul, das einen Nadelschafthalter (36, 42) und eine Ausrichtungsanordnung umfasst, die zwei Kugelgelenke (41, 43) umfasst, die verschiedene Schwenkpunkte für eine in dem Nadelschafthalter gehaltene Nadel bereitstellen, so dass sich kegelförmige Bewegungshüllkurven für die Nadel ergeben, und
ein Bildgebungsmodul, das eine Ultraschallsonde (23) hält und positioniert,
wobei das Nadelmodul und das Bildgebungsmodul fix an einer Trägerplattform (27) mit 5 Freiheitsgraden befestigt sind.

2. Nadelführungsvorrichtung nach Anspruch 1, worin das Nadelmodul ferner einen Vortriebsanschlag (58) umfasst, der eine gewünschte Nadeleindringtiefe festlegt und auf eine auf den Bildgebungsdaten von der Ultraschallsonde beruhende vorberechnete Position eingestellt ist.

3. Nadelführungsvorrichtung nach Anspruch 1, worin das Bildgebungsmodul einen Ultraschallsondenschaft (21), eine Ultraschallsonde und einen Ultraschallsondenhalter umfasst, der geeignet ist, um die Sonde unter Einsatz eines Motors zu verschieben.

## Revendications

1. Appareil de guidage d'aiguille pour guider une aiguille (38) dans un emplacement sélectionné d'un patient relativement à un instrument d'imagerie (23), pour des procédures d'intervention percutanées, comprenant:
un module à aiguille comprenant un support de gaine d'aiguille (36, 42) et un ensemble d'orientation qui comprend deux joints sphériques (41, 43) réalisant des points de pivotement différents pour une aiguille retenue dans le support de gaine d'aiguille et se traduisant par des enveloppes de mouvement coniques pour ladite aiguille; et
un module d'imagerie qui retient et positionne une sonde ultrasonique (23);
ledit module à aiguille et ledit module d'imagerie étant installés fixement sur une plate-forme de support 5 DOF (27).

2. Appareil de guidage d'aiguille selon la revendication 1, dans lequel le module à aiguille comprend en outre une butée d'arrêt de tir (58) qui règle une profondeur d'aiguille souhaitée et qui est réglée à une position pré-calculée basée sur les données d'imagerie de la sonde ultrasonore.

3. Appareil de guidage d'aiguille selon la revendication 1, dans lequel le module d'imagerie comporte une gaine de sonde ultrasonore (21) et une sonde ultrasonore, et un support de sonde ultrasonore apte à translater la sonde en utilisant un moteur.
